# FASCICULE DE BREVET EUROPEEN

(11) **EP 1 065 984 B1**
(45) Date de publication et mention de la délivrance du brevet: **19.11.2008**
(21) Numéro de dépôt: 99911850.8
(22) Date de dépôt: 30.03.1999
(51) Int. Cl.: A61B 17/70

(54) **DISPOSITIF DE CONNEXION POUR OSTEOSYNTHESE**
OSTEOSYNTHETISCHE VERBINDUNGSVORRICHTUNG
CONNECTING DEVICE FOR OSTEOSYNTHESIS

(30) Priorité: 31.03.1998 FR 9804257
(43) Date de publication de la demande: 10.01.2001
(73) Titulaire: Letendart, Joël, 59880 Saint Saulve (FR); Cordonnier, Denis, 59185 Provin (FR); Desrousseaux, Jean-François, 59274 Marquillies (FR); Stahl, Philippe, 59262 Sainghin en Melantois (FR); Mathevon, Henri, 59240 Dunkerque (FR)
(72) Inventeur: Letendart, Joël, 59880 Saint Saulve (FR); Cordonnier, Denis, 59185 Provin (FR); Desrousseaux, Jean-François, 59274 Marquillies (FR); Stahl, Philippe, 59262 Sainghin en Melantois (FR); Mathevon, Henri, 59240 Dunkerque (FR)
(74) Mandataire: Poncet, Jean-François
(86) Numéro de dépôt international: PCT/FR1999/000742
(87) Numéro de publication internationale: WO 1999/049802

(56) Documents cités:
- EP-A- 0 408 489
- EP-A- 0 729 731
- DE-A- 19 512 709
- FR-A- 2 692 471
- FR-A- 2 693 365
- FR-A- 2 730 155
- US-A- 5 312 404
- US-A- 5 474 551
- US-A- 5 613 968

## Description

La présente invention concerne un dispositif de connexion utilisable dans l'instrumentation chirurgicale du rachis. Elle concerne plus particulièrement un dispositif apte à réaliser la connexion entre des vis à visée pédiculaire ou articulaire et une tige de support vertébral destinée à maintenir la colonne vertébrale dans un alignement déterminé. L'instrumentation comportant les vis, le dispositif de connexion et la tige de support vertébral est utilisée notamment pour réaliser l'arthrodèse d'un segment du rachis.

On connaît par le document EP.0.425.783 un tel dispositif de connexion consistant en une pince unilatérale destinée à fixer une vis pédiculaire à une tige de support vertébral dont les axes centraux respectifs sont situés dans des plans différents. Il importe que l'inclinaison des deux axes, respectivement de la tige de support vertébral et de chaque vis pédiculaire, puisse varier de même que la distance entre ces deux axes pour pouvoir être parfaitement adaptée à la conformation souhaitée par le chirurgien.

S'agissant du document EP.0.425.783, la liberté d'inclinaison angulaire est donnée grâce à une rotule constituant soit la tête de la vis pédiculaire soit une extrémité de la pièce de connexion. Cette rotule doit obligatoirement être insérée dans un logement et bloquée en position dans ce logement une fois que l'inclinaison angulaire a été déterminée. Lorsque la rotule constitue la tête de la vis pédiculaire, le logement fait partie de la pièce de connexion et, dans les variantes de réalisation décrites dans ce document antérieur, est constitué par des évidements pratiqués dans deux parties distinctes de la pièce de connexion, parties qui sont articulées comme des mâchoires, le blocage de la rotule se faisant en rapprochant lesdites mâchoires l'une de l'autre. Ainsi lors de la mise en place de la vis pédiculaire, celle-ci doit obligatoirement être préalablement passée à travers la partie de la pièce de connexion qui fait office de mâchoire inférieure avant d'être insérée dans l'os. La présence de la pièce de connexion lors de l'implantation de la vis pédiculaire peut être une gêne pour le chirurgien.

Dans la variante de réalisation où la rotule consiste en une extrémité de la pièce de connexion, le logement est constitué par une portion évidée de la tête de la vis pédiculaire. Dans ce cas, lors de l'implantation de la vis pédiculaire, il importe que la tête de ladite vis soit orientée sensiblement dans la direction appropriée pour le passage de la tige de support vertébral, ce qui constitue une contrainte pour le chirurgien.

Par ailleurs, le document DE 195 12 709 décrit un dispositif d'ostéosynthèse comportant une vis à implanter, une pièce de connexion et des moyens de fixation de la pièce de connexion sur la vis à implanter. Ces moyens de fixation sont constitués de deux éléments comportant chacun une portion hémisphérique convexe. La vis à implanter comporte une embase hémisphérique concave et deux portions filetées, l'une destinée à être implantée dans l'os, l'autre destinée à son assemblage avec la pièce de connexion. La fixation de la pièce de connexion sur la vis à implanter est réalisée par serrage de la pièce de connexion entre les deux éléments formant les moyens de fixation. La partie convexe de chacun des éléments coopère respectivement avec l'embase concave de la tête de la vis à implanter et un écrou qui présente une portion concave et qui est vissée sur la portion filetée destinée à l'assemblage de la vis à implanter et de la pièce de connexion.

Contrairement au dispositif décrit dans le document EP. 0 425 783, ce dispositif permet de désolidariser la pièce de connexion et la vis facilitant ainsi la pose du dispositif.

De plus, les éléments formant les moyens de fixation de la pièce de connexion et de la vis comportant des portions hémisphériques de même centre, il est ainsi aisé de varier l'orientation angulaire des axes de la vis et de la tige de support sans prendre de précautions lors de l'implantation de la vis dans le rachis pour que cette dernière soit déjà au préalable orientée sensiblement dans la direction désirée.

Néanmoins, l'utilisation d'un tell dispositif multiplie les manipulations à effectuer par le chirurgien. En effet, celui-ci doit, après l'implantation de la vis dans le rachis, positionner correctement les éléments formant les moyens de fixation précédemment décrits ce qui allonge le temps de pose du dispositif. De plus, la présence de ces moyens de fixation augmente l'encombrement du dispositif de connexion ce qui n'est pas souhaitable pour le patient.

EP-A1-0 408 489 montre les caractéristiques du préambule de la revendication 1.

Le but que s'est fixé le demandeur est de proposer un dispositif de connexion qui pallie les inconvénients des dispositifs précédemment décrits.

Il s'agit d'un dispositif de connexion pour ostéosynthèse, destiné à relier une vis implantable dans le rachis à une tige de support vertébral, dont les axes centraux respectifs ne sont pas dans le même plan, ledit dispositif comprenant de manière connue une pièce de connexion, des moyens de blocage de la tige de support vertébral sur ladite pièce de connexion, la vis à implanter étant constituée d'une première portion filetée inférieure pour son implantation dans les rachis, d'une tête à embase courbe et d'une seconde portion filetée supérieure dans le prolongement de la première, la pièce de connexion présentant pour la connexion avec la vis un trou dont le diamètre intérieur est plus grand que le diamètre extérieur de la seconde portion filetée.

De manière caractéristique, selon l'invention, la tête de la vis est à pans et le dispositif comprend également un écrou à embase courbe qui est apte à coopérer avec la seconde portion filetée de la vis ; le blocage en position de la vis sur la pièce de connexion étant obtenu, de façon caractéristique, après vissage de l'écrou, par pincement de l'embase courbe de l'écrou sur la face d'appui supérieure de la pièce de connexion et pincement de la face d'appui inférieure de la pièce de connexion sur l'embase courbe de la tête de la vis, les surfaces courbes des embases de la tête de la vis, de l'écrou de blocage et des faces d'appui supérieure et inférieure de la pièce de connexion étant des portions de sphères dont la position des centres est identique, l'une des faces d'appui supérieure et inférieure étant concave, l'autre tant convexe.

Lors de la mise en place de l'instrumentation, le chirurgien peut implanter sans contrainte toutes les vis pédiculaires à l'aide d'une clé à pan, sans soucis d'orientation desdites vis. La mise en place de chaque pièce de connexion intervient par l'introduction de la tige filetée supérieure au travers du trou à assise courbe, ladite tige filetée servant de tige de guidage pour le placement de ladite pièce de connexion.

Les surfaces d'appui supérieure et inférieure de la pièce de connexion étant des portions de sphères concentriques, le réglage angulaire entre la vis et la tige de support vertébral est facile à mettre en oeuvre et permet une variation angulaire quasi infinie des positions respectives de ces deux organes. De plus, on obtient ainsi un blocage en position particulièrement efficace du fait que le coincement des différents organes entre eux est réalisé, quelle que soit leur position relative, par l'application l'une contre l'autre de zones surfaciques.

Selon un mode préféré de réalisation, les moyens de blocage de la tige de support vertébral sur la pièce de connexion comportent :
a) une pièce de blocage présentant un premier passage pour la tige de support vertébral, un deuxième passage pour une partie de la pièce de connexion, le premier et le deuxième passages étant sensiblement perpendiculaires et ayant un volume intérieur commun dans lequel la tige et la plaque de connexion peuvent être en contact, et un troisième passage fileté débouchant dans le volume intérieur du premier ou du deuxième passages et,
b) un bouchon de blocage fileté apte à se déplacer par vissage dans le troisième passage et à bloquer en position la tige de support vertébral et la plaque de connexion par pincement du bouchon, de la tige, de la plaque et de la pièce les uns contre les autres.

La présence de la pièce de blocage coulissant sur la pièce de connexion permet de régler l'écartement entre la vis à implanter et la tige de support vertébral.

Dans cette configuration, la pièce de connexion ne comporte pas une fente comme dans le document EP.0.425.783. La partie de la pièce de connexion qui est destinée à coulisser dans le deuxième passage de la pièce de blocage peut avoir toutes configurations ; de préférence il s'agit d'une plaque de section sensiblement rectangulaire à bords arrondis, le second passage étant conformé pour permettre le coulissement ajusté de la pièce de blocage sur la partie de la pièce de connexion allant au-delà des faces d'assise courbes. La forme rectangulaire de la section de cette partie de la pièce de connexion permet également de diminuer l'encombrement de la pièce de blocage, comparativement à une partie de section circulaire de même résistance mécanique.

De même, cette configuration permet de fixer la pièce de connexion sur la vis à implanter puis, lorsque cette opération est effectuée, de régler l'écartement de la tige de support vertébral et de la vis à implanter sans action sur le mode de fixation de la pièce de connexion sur la vis et donc sans risque de désolidariser celles-ci ou de modifier leurs positions respectives.

En effet, dans le document DE.195 12 709, le réglage de l'écartement de la vis et de la tige de support vertébral s'effectue simultanément, lors de la fixation de la pièce de connexion sur la vis par positionnement de la seconde tige filetée dans le trou de la pièce de connexion qui présente un diamètre supérieur à celui de cette dernière.

De préférence le premier passage, pour la tige de support vertébral, a une section transversale de forme oblongue, autorisant un certain déplacement en hauteur de la tige avant le vissage du bouchon de blocage et le passage d'une tige préalablement cintrée.

Selon une version préférée de réalisation, la pièce de blocage est une pièce cylindrique, les premier et deuxième passages ont des directions diamétrales, perpendiculaires l'une à l'autre et le troisième passage est formé dans une des deux faces circulaires du cylindres, débouchant dans le volume intérieur du premier passage.

Avantageusement la pièce de connexion comporte un élément formant butée ou est conformée en sorte de pouvoir elle-même constituer une butée pour la pièce de blocage lors du libre coulissement de celle-ci, avant le vissage du bouchon de blocage.

Avantageusement la seconde partie filetée supérieure de la vis pédiculaire est sécable, présentant une zone de fragilisation. Selon cette disposition particulière, après vissage de l'écrou à embase courbe assurant le blocage en position de la vis pédiculaire sur la pièce de connexion, il est possible d'éliminer la partie haute de la seconde portion filetée de la vis pédiculaire, qui dépasse de l'écrou de manière à réduire au maximum l'encombrement de l'instrumentation.

La présente invention sera mieux comprise à la lecture de la description qui va être faite d'un exemple préféré de réalisation du dispositif de connexion pour ostéosynthèse destiné à relier une vis implantable dans le rachis à une tige de support vertébral, illustré par le dessin annexé dans lequel :
- La figure 1 est une représentation schématique en perspective du dispositif de connexion,

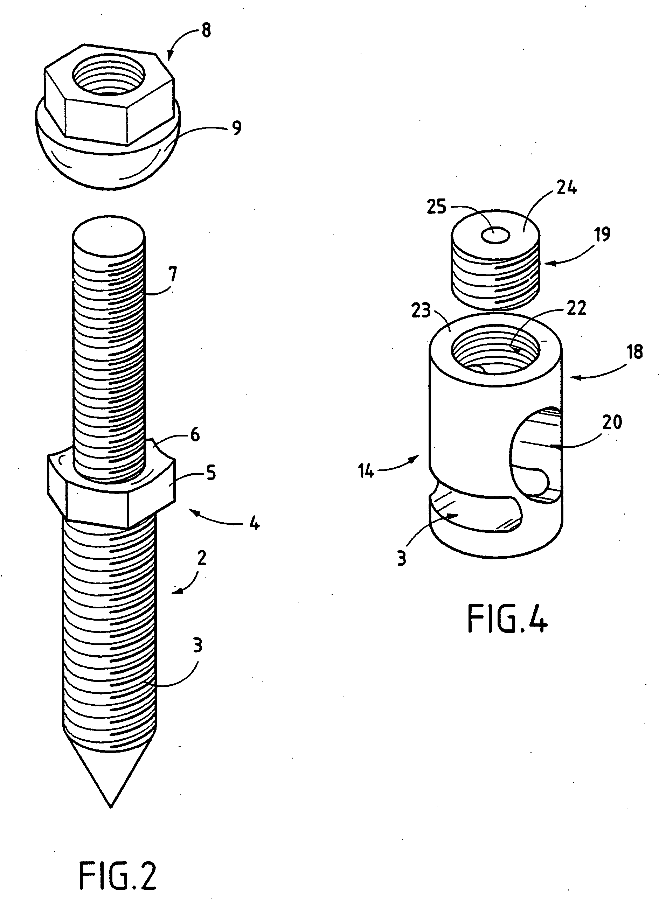

- La figure 2 est une représentation schématique en perspective de la vis pédiculaire et de l'écrou à embase courbe,
- La figure 3 est une représentation schématique en perspective de la plaque de connexion et
- La figure 4 est une représentation schématique en perspective de la pièce de blocage et de son bouchon de blocage,
- Les figures 5 et 6 sont des représentations schématiques en coupe longitudinale du dispositif au niveau de la connexion entre la vis pédiculaire, la plaque de connexion et l'écrou à embase courbe dans deux positions angulaires différentes.

L'arthrodèse est une technique chirurgicale consistant à fusionner les deux os d'une articulation malade afin d'en supprimer le mouvement relatif. S'agissant d'une arthrodèse rachidienne, cette technique conduit à assurer la fusion de deux ou de plusieurs vertèbres voisines entre-elles. Elle nécessite une fixation temporaire, voire même définitive, desdites vertèbres grâce à une instrumentation chirurgicale appropriée. Cette instrumentation comporte de manière connue des vis à visée pédiculaire ou articulaire, une tige de support vertébral qui est généralement une tige métallique de grande rigidité qui est destinée à maintenir le segment du rachis concerné dans une configuration déterminée. Cette instrumentation comporte également des moyens de connexion réalisant le blocage en position des vis pédiculaires par rapport à la tige de support vertébral. Une telle instrumentation est connue notamment par le document EP.0.425.783.

Selon l'invention, la vis pédiculaire 2 présente une structure particulière, avec une première portion inférieure filetée 3, surmontée d'une tête 4 à pans 5 et à embases 6 courbes et d'une seconde portion filetée supérieure 7, dans le prolongement de la première portion filetée inférieure 3 par exemple la tête 4 est une tête à six pans 5.

Un écrou 8 à embase 9 courbe est adapté pour se déplacer par vissage sur la seconde portion filetée supérieure 7 de la vis pédiculaire 2. Il s'agit par exemple d'un écrou à six pans coopérant avec une tige filetée 7 de l'ordre de 5 mm de diamètre.

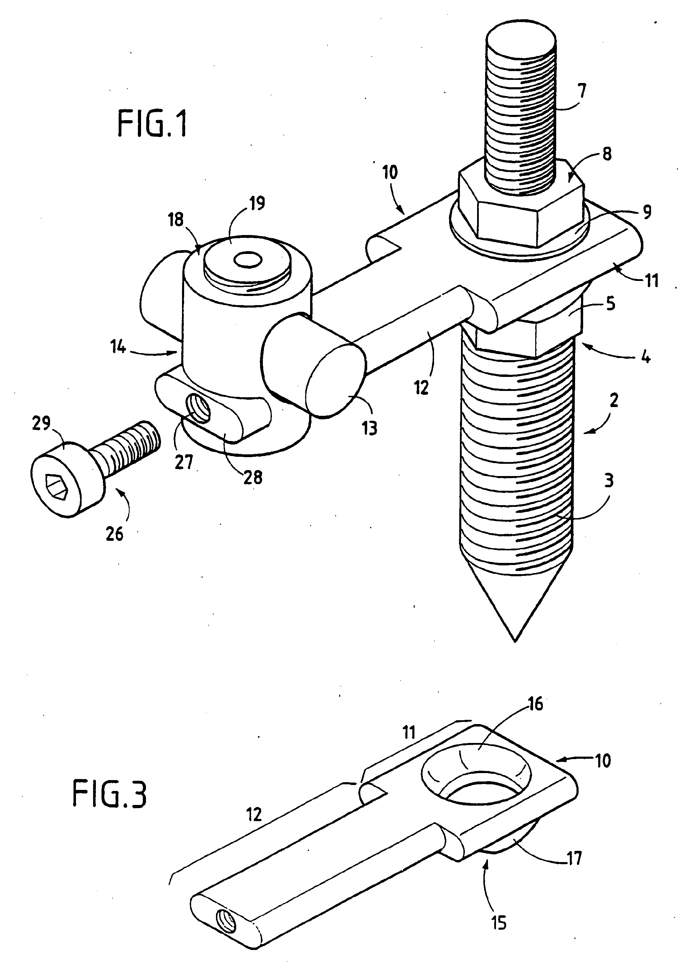

Le dispositif 1 de connexion comporte une plaque 10 de connexion, telle qu'illustrée à la figure 3. Cette plaque 10 présente deux parties 11, 12, la première destinée à permettre la connexion avec la vis pédiculaire 2 grâce à l'écrou 8 et la seconde 12 destinée à assurer la connexion avec la tige 13 de support vertébral grâce à l'ensemble de blocage 14.

La première partie 11 de la plaque de connexion présente une structure en forme de cratère dans laquelle un trou 15 est bordé de deux faces d'appui, une face supérieure concave 16 bordant la partie en creux du cratère et une face inférieure convexe 17. Les deux faces d'appui 16,17 ont exactement la même configuration courbe que les embases 6 et 9 d'une part de la tête 4 de la vis pédiculaire 2 et de l'écrou 8. De plus le diamètre intérieur du trou 15 de la plaque de connexion 10 est supérieur au diamètre extérieur de la seconde portion filetée 7 de la vis pédiculaire 2. Dans un exemple de réalisation, dans lequel la seconde portion filetée 7 de la vis 2 faisait de l'ordre de 5mm, le diamètre intérieur du trou 15 était de l'ordre de 7mm.

La seconde partie 12 de la plaque de connexion 10 prolonge la première partie 11 ; elle a une section sensiblement rectangulaire dont les bords sont arrondis de manière à éviter les arêtes vives.

L'élément de blocage 14 est composé d'une pièce de blocage cylindrique 18 et d'un bouchon de blocage 19. La pièce cylindrique 18 est traversée de part en part, diamétralement, par un premier passage 20, destiné à permettre l'introduction et le coulissement de la tige 13 de support vertébral et un second passage 21 diamétral et perpendiculaire au premier passage 20 destiné à permettre l'introduction et le coulissement de la seconde partie 12 de la plaque de connexion 10. Le perçage des deux passages 20,21 est réalisé en sorte que le volume intérieur des deux, passages se recoupe ; ainsi la tige 13 de support vertébral peut être en contact avec la seconde partie 12 de la plaque de connexion 10, lorsque ces deux éléments sont introduits dans la pièce 18 de blocage.

La pièce 18 de blocage comporte un troisième passage 22 qui est percé dans la face circulaire 23 de la pièce cylindrique 18 et qui débouche dans le volume intérieur du premier passage 20 à l'opposé du deuxième passage 21.

Le bouchon 19 de blocage a sa périphérie filetée étant apte à se déplacer par vissage à l'intérieur du troisième passage 22 également fileté dans la face supérieure 24 du bouchon 19 est prévue une empreinte en creux 25 pour l'introduction d'un outil de vissage.

La mise en oeuvre du dispositif 1 de connexion sur le segment rachidien à arthrodéser est réalisée dans les conditions suivantes. On implante tout d'abord les vis pédiculaires 2 ou articulaires. On glisse ensuite, sur chaque vis 2, une plaque de connexion 10 en introduisant la seconde portion filetée de la vis 2 dans le trou 15 de la plaque 10 jusqu'à ce que la face d'assise inférieure 17 soit en appui sur l'embase courbe 6 de la tête 4 de la vis 2. On commence à visser sur la seconde portion filetée 7 de chaque vis 2 un écrou 8 pour tenir en place la plaque de connexion 10 correspondante mais sans la bloquer en position. On introduit sur chaque plaque de connexion 10 une pièce de blocage 14 en faisant pénétrer la seconde partie 12 de ladite plaque 10 dans le second passage 21 de la pièce 18.

On introduit ensuite une tige 13 de support vertébral successivement dans chaque premier passage 20 des pièces 18 de blocage adjacentes sur le tracé du segment rachidien.

La connexion par les faces d'appui courbes 16,17 de la pièce de connexion 10, et par les embases courbes 6 de la tête 4 de la vis 2 et 9 de l'écrou 8 ainsi que la possibilité de coulissement des pièces 18 de blocage le long des plaques de connexion 10 permet d'assurer au montage une mobilité interne en translation et rotation suivant toutes les directions possibles.

Avant de fixer grâce aux écrous 8 et au bouchon 19 l'ensemble des éléments du dispositif 1, le chirurgien peut s'assurer de la bonne relation de cet ensemble par des actions de contournage de la tige 13 de support vertébral ou en dérotant celle-ci. Ces différentes opérations, introduction et contournage de la tige 13 sont facilitées par le fait de la mobilité des différentes connexions.

Grâce à la forme sphérique et à la concentricité des faces d'appui 16,17 et des embases 6,9, le pincement des différents éléments entre-eux s'effectue selon des surfaces de contact, ce qui procure une très grande solidité à l'instrumentation.

Du fait du contact direct de la seconde partie 12 de la plaque de connexion 10 et de la tige 13 de support vertébral dans leur passage respectif 21,20, on obtient un blocage de ceux deux éléments avec la pièce 18 grâce au vissage du seul bouchon 19 de blocage dans son passage 22.

De manière à limiter encore plus l'encombrement de cette instrumentation, de préférence la seconde portion 7 filetée de la vis 2 est sécable à une hauteur un peu au-dessus de l'écrou 8 lorsqu'il est en position de blocage contre la plaque de connexion 10. Ce caractère sécable peut être obtenu en fragilisant cette portion de la tige par une ou plusieurs entailles permettant une cassure franche en exerçant, à l'aide d'un outil approprié, une force de flexion suffisante.

Pour réduire cet encombrement, de préférence, la pièce cylindrique 18 de blocage a un diamètre extérieur qui est sensiblement de la même largeur que la première partie 11 de la plaque de connexion 10. Dans ce cas, comme illustré à la figure 1, la seconde partie 12 de ladite plaque 10 a une largeur qui est inférieure à celle de la première partie 11.

Il peut être opportun d'empêcher le libre coulissement de la pièce cylindrique 18 de blocage lorsque celle-ci a été introduite sur la plaque de connexion 10 et avant qu'elle n'ait été fixée à celle-ci grâce au bouchon 19 de blocage . Ceci peut être obtenu par tout moyen approprié. Sur la figure 1, on a représenté à titre d'exemple une vis 26 apte à être vissée dans un trou 27 taraudé et percé dans la tranche 28 de la seconde partie 12 de la plaque de connexion 10. Cette vis 26 a une tête 29 qui a une dimension extérieure plus grande que ladite seconde portion 12. La vis 26 est mise en place après l'introduction de la pièce de blocage 18 sur la plaque de connexion 10, de sorte que la tête 29 de cette vis 26 sert de butée et empêche la pièce de blocage 28 de sortir de la plaque de connexion 10.

Le même effet de butée peut être obtenu en créant vers l'extrémité de l'extrémité de la seconde portion 12 proche de la tranche 28 de la plaque de connexion 10 une irrégularité de surface suffisante pour empêcher le libre coulissement de la pièce de blocage 18 . Cette irrégularité peut être créée au moyen d'un outil du type pince apte à déformer localement le matériau constitutif de la plaque de connexion 10.

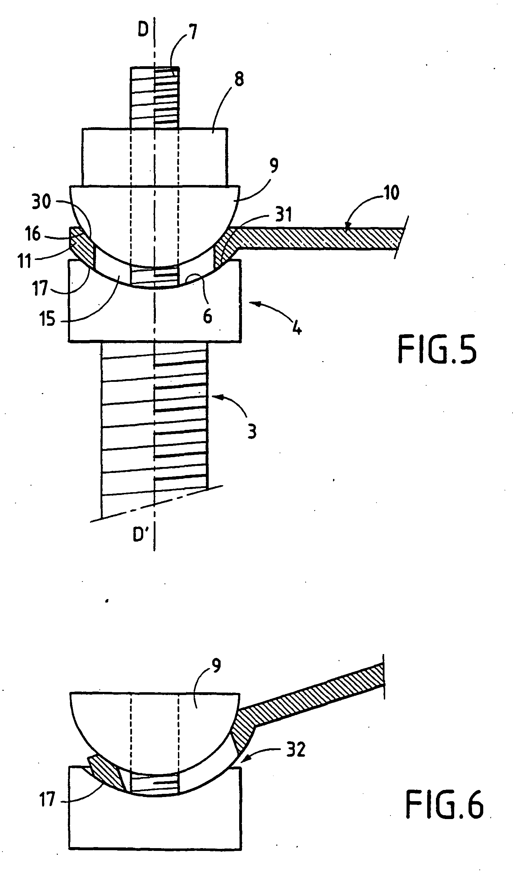

Pour bien faire comprendre la faculté de déplacement angulaire dans toutes les directions de la plaque de connexion 10 par rapport à la vis pédiculaire 2, on a représenté aux figures 5 et 6, en coupe longitudinale, partiellement la plaque de connexion 10, à l'écrou 8 et son embase courbe 9 ainsi que la vis pédiculaire 2 avec les deux portions filetées 3, 7, la tête 4 et son embase courbe 6 . Les différentes courbes sont concordantes de manière à ce que le contact puisse se faire sur des surfaces, quel que soit le déplacement relatif de la plaque 10.

A la figure 5, le trou 15 de la plaque de connexion 10 est centré par rapport à l'axe longitudinal DD' des portions filetées 3 et 7 de la vis 2. Le contact entre la face d'appui supérieure 16 de la plaque de connexion 10 et l'embase courbe 9 de l'écrou 8 se fait selon une zone 30, annulaire sphérique de révolution autour de l'axe DD'. De même le contact entre la face d'appui inférieure 17 de la plaque 10 et l'embase courbe 6 de la tête 4 de la vis 2 se fait selon une zone 31 annulaire sphérique de révolution autour de l'axe DD'.

Tant que l'écrou 8 n'est pas serré contre la plaque 10, celle-ci peut se déplacer dans tous les sens possibles entre les deux embases courbes 6 et 9 de la tête 4 et de l'écrou 8. Ce déplacement est limité uniquement par la dimension de la seconde portion filetée 7 de la vis et du trou 15. En effet, comme représenté à la figure 6, la seconde portion filetée 7 de la vis 2 constitue une butée pour la plaque 10 et limite ainsi son déplacement angulaire.

Les dimensions de cette seconde portion filetée 7 et du trou 15 sont déterminées en sorte d'obtenir les déplacements angulaires nécessaires lors de la mise en place du dispositif 1.

On peut remarquer, dans l'exemple illustré à la figure 6, que le contact entré la face d'appui inférieure 17 et l'embase courbe 6 de la tête 4 de la vis 2 ne se fait plus selon une zone de révolution. En l'espèce, il existe même une zone 32 où il n'y a plus d'appui entre les deux éléments Cependant cette absence localisée d'appui est compensée par une plus grande surface de contact à d'autres emplacements, ce qui assure une solidité très importante de l'instrumentation après serrage de l'écrou 8.

La présente invention n'est pas limitée au mode de réalisation qui vient d'être décrit à titre d'exemple non exhaustif. En particulier l'embase de l'écrou et l'embase de la tête de la vis pédiculaire peuvent être de forme inversée par rapport à l'exemple décrit et illustré, étant en creux dans l'écrou et proéminent dans la tête de la vis.

## Revendications

1. Dispositif de connexion (1) pour ostéosynthèse, destiné à relier une vis (2) implantable dans le rachis à une tige (13) de support vertébral, dont les axes centraux respectifs ne sont pas dans le même plan, comprenant une pièce de connexion (10), des moyens de blocage de la tige de support vertébral sur ladite pièce, la vis étant constituée d'une première portion filetée (3) inférieure pour l'implantation dans les rachis, d'une tête (4) à embase courbe (6) et une seconde portion filetée supérieure (7) dans le prolongement de la première (3), la pièce de connexion (10) présentant, pour la connexion avec la vis (2), un trou (15) dont le diamètre intérieur est plus grand que le diamètre extérieur de la seconde portion filetée (7) de la vis (2), **caractérisé en ce que** la tête (4) de la vis est à pans (5), **en ce qu'**il comprend également un écrou (8) à embase courbe (9) qui est apte à coopérer avec la seconde portion filetée (7) et **en ce que** le blocage en position de la vis (2) sur la pièce de connexion (10) est obtenu, après vissage de l'écrou (8), par pincement de l'embase courbe (9) de l'écrou (8) sur la face d'appui supérieure (16) de la pièce de connexion (10) et pincement de la face d'appui inférieure (17) de la pièce de connexion (10) sur l'embase courbe (6) de la tête (4) de la vis (2), les surfaces courbes des embases (6,9) de la tête (4) de la vis (2), de l'écrou (8) de blocage et des faces d'appui supérieure (16) et inférieure (17) de la pièce de connexion (10) sont des portions de sphères dont la position des centres est identique, l'une des deux faces d'appui supérieure et inférieure (16,17) étant convexe, l'autre étant concave, en sorte de permettre la rotation de la pièce de connexion (10) par rapport à la vis (2).

2. Dispositif de connexion selon la revendication 1 **caractérisé en ce que** les moyens de blocage (14) de la pièce de connexion (10) sur la tige (13) de support vertébral comportent :
a) une pièce de blocage (18) présentant un premier passage (20) pour la tige (13) de support vertébral, un deuxième passage (21) pour une partie (12) de la pièce de connexion (10), le premier (20) et le deuxième (21) passages étant sensiblement perpendiculaires et ayant un volume intérieur commun dans lequel la tige (13) et la pièce de connexion (10) peuvent être en contact, et un troisième passage (22) fileté débouchant dans le volume intérieur du premier (20) ou du deuxième (21) passage et,
b) un bouchon de blocage (19) fileté apte à se déplacer par vissage dans le troisième passage (22) et à bloquer en position la tige (13) de support vertébral et la pièce de connexion (10) par pincement du bouchon (19), de la tige (13), de la pièce (10) et de la pièce (18) les uns contre les autres.

3. Dispositif de connexion selon la revendication 2 **caractérisé en ce que** la partie (12) de la pièce de connexion (10) qui est destinée à coulisser dans le deuxième passage (21) de la pièce de blocage (18) est une plaque de section sensiblement rectangulaire à bords arrondis, le second passage (21) étant conformé pour permettre le coulissement ajusté de la pièce de blocage (18) sur ladite partie (12) de la pièce de connexion (10) allant au-delà des faces d'appui courbes (16,11).

4. Dispositif de connexion selon l'une des revendications 2 ou 3 **caractérisé en ce que** le premier passage (20), pour la tige (13) de support vertébral, a une section transversale de forme oblongue, autorisant un certain déplacement en hauteur de la tige (13) avant le vissage du bouchon de blocage (19) et le passage d'une tige préalablement cintrée.

5. Dispositif de connexion selon l'une des revendications 2 à 4 **caractérisé en ce que** la pièce de blocage (18) est une pièce cylindrique, les premier. (20) et deuxième (21) passages ont des directions diamétrales, perpendiculaires l'une à l'autre et le troisième passage (22) est formé dans une (24) des deux faces circulaires du cylindre, débouchant dans le volume intérieur du premier passage (20).

6. Dispositif de connexion selon l'une des revendications 2 à 5 **caractérisé en ce que** la pièce de connexion (10) comporte un élément formant butée ou est conformée en sorte de pouvoir elle-même constituer une butée pour la pièce de blocage (18) lors du libre coulissement de celle-ci, avant le vissage du bouchon de blocage (19).

7. Dispositif de connexion selon l'une quelconque des revendications 1 à 6 **caractérisé en ce que** la seconde partie filetée supérieure (7) de la vis (2) est sécable, présentant une zone de fragilisation.

## Claims

1. Connection device (1) for osteosynthesis, intended to connect a screw (2) that can be implanted in the spine and a vertebral support rod (13), the respective central axes whereof are not in the same plane, comprising a connecting piece (10), means for locking the vertebral support rod to said piece, the screw consisting of a first threaded portion (3) at the bottom for implantation in the spine, a head (4) with a curved base (6), and a second threaded portion (7) at the top in line with the first threaded portion (3), the connecting piece (10) having, for the connection with the screw (2), a hole (15) the inside diameter whereof is greater than the outside diameter of the second treaded portion (7) of the screw (2), **characterised in that** the head (4) of the screw has flats (5), **in that** it further comprises a nut (8) with a curved base (9) that is adapted to cooperate with the second threaded portion (7), and **in that** the screw (2) is locked in position on the connecting piece (10), after the nut (8) has been screwed, by clamping the curved base (9) of the nut (8) to the top bearing face (16) of the connecting piece (10) and by clamping the bottom bearing face (17) of the connecting piece (10) to the curved base (6) of the head (4) of the screw (2), the curved surfaces of the bases (6, 9) of the head (4) of the screw (2), of the locking nut (8) and of the top bearing face (16) and bottom bearing face (17) of the connecting piece (10) are portions of spheres with centres identically positioned, one of the top and bottom bearing faces (16, 17) being convex and the other being concave, to allow rotation of the connecting piece (10) relative to the screw (2).

2. Connecting device according to claim 1 **characterised in that** the means (14) for locking the connecting piece (10) to the vertebral support rod (13) include:
a) a locking piece (18) having a first passage (20) for the vertebral support rod (13), a second passage (21) for a portion (12) of the connecting piece (10), the first passage (20) and the second passage (21) being substantially perpendicular and having a common interior volume in which the screw (13) and the connecting piece (10) can be in contact, and a threaded third passage (22) opening into the interior volume of the first passage (20) or the second passage (21), and
b) a threaded locking plug (19) adapted to be screwed into the third passage (22) and to lock the vertebral support rod (13) and the connecting piece (10) in position by clamping together the plug (19), the rod (13), the piece (10) and the piece (18).

3. Connecting device according to claim 2 **characterised in that** the portion (12) of the connecting piece (10) intended to slide in the second passage (21) in the locking piece (18) is a plate of substantially rectangular section with rounded edges, the second passage (21) being shaped to allow snug sliding of the locking piece (18) on said portion (12) of the connecting piece (10) extending beyond the curved bearing faces (16, 17).

4. Connecting device according to either of claims 2 or 3 **characterised in that** the first passage (20) for the vertebral support rod (13), has an oblong cross section allowing some movement of the rod (13) in the heightwise direction before screwing in the locking plug (19) and passing through a previously curved rod.

5. Connecting device according to any one of claims 2 to 4 **characterised in that** the locking piece (18) is a cylindrical piece, the first passage (20) and the second passage (21) have diametral directions, perpendicular to each other, and the third passage (22) is formed in one circular face (24) of the two circular faces of the cylinder opening into the interior volume of the first passage (20).

6. Connecting device according to any one of claims 2 to 5, **characterised in that** the connecting piece (10) includes an abutment member or is shaped so as of itself to constitute an abutment for the locking piece (18) upon free sliding thereof, before the locking plug (19) is screwed in.

7. Connecting device according to any one of claims 1 to 6, **characterised in that** the second threaded portion (7) at the top of the screw (2) is suitable for being broken off, including a weakened area.

## Patentansprüche

1. Verbindungsvorrichtung (1) für Osteosynthese, die dazu bestimmt ist, eine in das Rückgrad implantierbare Schraube (2) mit einem vertebralen Stützschaft (13) zu verbinden, deren entsprechende zentrale Achsen nicht in derselben Ebene sind, mit einem Verbindungsteil (10), Mitteln zum Blockieren des vertebralen Stützschaftes an dem genannten Teil, wobei die Schraube durch einen ersten unteren Gewindeabschnitt (3) zur Implantation in die Wirbelsäule, einen Kopf (4) mit gekrümmter Basis (6) und einen zweiten oberen Gewindeabschnitt (7) in der Verlängerung der ersten (3) gebildet ist, wobei das Verbindungsteil (10) zur Verbindung mit der Schraube (2) ein Loch (15) aufweist, dessen innerer Durchmesser größer ist als der äußere Durchmesser des zweiten Gewindeabschnittes (7) der Schraube (2), **dadurch gekennzeichnet, dass** der Kopf (4) der Schraube flächig (5) ist, dass er auch eine Mutter (8) mit gekrümmter Basis (9) enthält, die geeignet ist, mit dem zweiten Gewindeabschnitt (7) zusammenzuwirken und dass das Blockieren der Schraube (2) in Position auf dem Verbindungsteil (10) nach Verschrauben der Mutter (8) durch Klemmen der gekrümmten Basis (9) der Mutter (8) an der oberen Anlagefläche (16) des Verbindungsteiles (10) und durch Klemmen der unteren Anlagefläche (17) des Verbindungsteiles (10) an der gekrümmten Basis (6) des Kopfes (4) der Schraube (2) erhalten wird, wobei die gekrümmten Flächen der Basen (6, 9) des Kopfes (4) der Schraube (2), der Mutter (8) zum Blockieren und der oberen (16) und unteren (17) Anlageflächen des Verbindungsteiles (10) sphärische Teile sind, deren Position der Zentren identisch ist, wobei eine der beiden oberen und unteren (16, 17) Anlageflächen konvex ist, wobei die andere konkav ist, derart, dass die Drehung des Verbindungsteiles (10) in Bezug auf die Schraube (2) erlaubt ist.

2. Verbindungsvorrichtung nach Anspruch 1, **dadurch gekennzeichnet, dass** die Mittel (14) zum Blockieren des Verbindungsteiles (10) an dem vertebralen Stützschaft (13) enthalten:
a) ein Blockierungsteil (18), das eine erste Passage (20) für den vertebralen Stützschaft (13) aufweist, eine zweite Passage (21) für einen Teil (12) des Verbindungsteils (10), wobei die erste (20) und die zweite (21) Passage im wesentlichen rechtwinklig sind und ein gemeinsames inneres Volumen haben, in welchem der Schaft (13) und das Verbindungsteil (10) in Kontakt sein können, und eine dritte Passage (22) mit einem Gewinde, die in das innere Volumen der ersten (20) oder der zweiten (21) Passage mündet, und
b) einen Blockierungsstopfen (19) mit Gewinde, der geeignet ist, sich durch Einschrauben in die dritte Passage (22) zu verschieben und den vertebralen Stützschaft (13) und das Verbindungsteil (10) in Position zu blockieren, durch Klemmen, des Stopfens (19), des Schaftes (13) des Teiles (10) und des Teiles (18) gegeneinander.

3. Verbindungsvorrichtung nach Anspruch 2, **dadurch gekennzeichnet, dass** der Abschnitt (12) des Verbindungsteiles (10), der dazu bestimmt ist, in der zweiten Passage (21) des Blockierungsteiles (18) zu gleiten, eine Platte mit im wesentlichen rechteckigem Querschnitt mit gerundeten Kanten ist, wobei die zweite Passage (21) ausgebildet ist, um ein justiertes Gleiten des Blockierungsteiles (18) auf dem genannten Teil (12) des Verbindungsteiles (10) über die gekrümmten Anlageflächen (16, 17) hinausgehend zu gestatten.

4. Verbindungsvorrichtung nach einem der Ansprüche 2 oder 3, **dadurch gekennzeichnet, dass** die erste Passage (20) für den vertebralen Stützschaft (13) einen Querschnitt mit langgestreckter Form hat, der eine gewisse Höhenverschiebung des Schaftes (13) vor dem Einschrauben des Blockierungsstopfens (19) und die Passage eines zuvor gebogenen Schaftes gestattet.

5. Verbindungsvorrichtung nach einem der Ansprüche 2 bis 4, **dadurch gekennzeichnet, dass** das Blockierungsteil (18) ein zylindrisches Teil ist, wobei die erste (20) und zweite (21) Passage diametrale Richtungen haben, die senkrecht zueinander stehen, und die dritte Passage (22) in einer (24) der zwei kreisförmigen Flächen des Zylinders geformt ist, der in das innere Volumen der ersten Passage (20) mündet.

6. Verbindungsvorrichtung nach einem der Ansprüche 2 bis 5, **dadurch gekennzeichnet, dass** das Verbindungsteil (10) ein Element trägt, das einen Anschlag bildet oder derart ausgebildet ist, dass es selbst einen Anschlag für das Blockierungsteil (18) während des freien Gleitens desselben vor dem Verschrauben des Blockierungsstopfens (19) bildet.

7. Verbindungsvorrichtung nach irgendeinem der Ansprüche 1 bis 6, **dadurch gekennzeichnet, dass** das zweite obere Gewindeteil (7) der Schraube (2) teilbar ist, indem es eine Schwächungszone aufweist.
